# EUROPÄISCHE PATENTANMELDUNG

(11) **EP 2 105 130 A1**
(43) Veröffentlichungstag der Anmeldung: **30.09.2009**
(21) Anmeldenummer: 08005531.2
(22) Anmeldetag: 25.03.2008
(51) Int. Cl.: A61K 9/14, A61K 31/415

(54) **Pharmazeutische Formulierung und Verfahren zu deren Herstellung**

(71) Anmelder: Ratiopharm GmbH, 89079 Ulm (DE)
(72) Erfinder: Kraahs, Peter Dr., 79189 Bad Krozimgen (DE); Schulze-Nahrup, Julia, Dr., 82061 Neuried (DE); Alles, Rainer Dr., 4125 Riehen (DE); Albers, Jessica, 45149 Essen (DE); Kleinbudde, Peter Dr., 40627 Düsseldorf (DE); Knop, Klaus Dr., 40764 Langenfeld (DE)
(74) Vertreter: Teipel, Stephan

(57) **Zusammenfassung**

Die vorliegende Erfindung betrifft eine pharmazeutische Formulierung, die eine feste Dispersion, die einen in amorpher Form in ein Polymer eingebetteten Wirkstoff enthält, und unabhängig von der festen Dispersion ein externes Polymer als Rekristallisationsinhibitor umfasst.

## Beschreibung

Die vorliegende Erfindung betrifft eine pharmazeutische Formulierung, die eine feste Dispersion, die einen in amorpher Form in ein Polymer eingebetteten Wirkstoff enthält, und unabhängig von der festen Dispersion ein externes Polymer als Rekristallisationsinhibitor umfasst.

Zahlreiche Versuche wurden unternommen, um die Bioverfügbarkeit von Wirkstoffen, insbesondere schwer löslichen Wirkstoffen zu erhöhen. Eine Möglichkeit besteht darin, entsprechende Wirkstoffe in amorpher Form zur Verfügung zu stellen. Verbindungen, die in amorpher statt kristalliner Form vorliegen, sind in der Regel leichter löslich und verfügen dementsprechend auch über eine höhere Bioverfügbarkeit.

Bei pharmazeutischen Formulierungen, die den Wirkstoff in amorpher Form enthalten, besteht allerdings das Problem, dass der Wirkstoff oftmals dazu neigt, während der Herstellung der Formulierung oder deren Lagerung auszukristallisieren. Verschiedene Versuche wurden unternommen, den Wirkstoff in amorpher Form zur Verfügung zu stellen und dann ein entsprechendes Rekristallisieren des Wirkstoffs zu unterdrücken. Eine der bekannten Möglichkeiten besteht darin, den Wirkstoff in amorpher Form in eine feste Dispersion eines Polymers als Löslichkeitsverbesserer einzubetten. Hierdurch können glasartige Partikel erhalten werden, in denen der Wirkstoff in amorpher Form vorliegt und die, wenn sie einen Rekristallisationsinhibitor enthalten, über einen längeren Zeitraum stabil sind, d.h. in denen der Wirkstoff zumindest während der üblichen Lagerdauer nicht rekristallisiert. Entsprechende feste Dispersionen sowie verschiedene Verfahren zu deren Herstellung sind beispielsweise aus der WO 03/000294 A1 sowie der EP-A-1 027 886 bekannt.

Wird die Löslichkeit eines an sich schwer löslichen Wirkstoffs beispielsweise durch die vorstehend beschriebenen Maßnahmen verbessert, so ergibt sich das weitere Problem, dass es durch die erhöhte Auflösungsgeschwindigkeit beispielsweise nach Verabreichung der pharmazeutischen Formulierung im Magen zu einer übersättigten Lösung des Wirkstoffs kommen kann. Aus einer derartigen, übersättigten Lösung kann der Wirkstoff dann wiederum in kristalliner Form ausfallen, wodurch die Bioverfügbarkeit des Wirkstoffs entsprechend sinkt. Es ist bekannt, dass dieser nachteilige Effekt aufgrund der Löslichkeitsverbesserung bei einem an sich schwer löslichen Wirkstoff dadurch abgemildert werden kann, dass die pharmazeutische Formulierung zusätzlich einen Lösungsstabilisator enthält, der die übersättigte Lösung des Wirkstoffs stabilisiert und damit ein Rekristallisieren des Wirkstoffs verhindert. Entsprechende pharmazeutische Formulierungen sind beispielsweise in der WO 03/000294 A1 und der WO 03/000235 A1 beschrieben. Diese Dokumente beschreiben auch, dass der Lösungsstabilisator entweder Teil der festen Dispersion sein kann, als Gemisch zusammen mit der festen Dispersion in der pharmazeutischen Formulierung vorliegen kann oder im zeitlichen Zusammenhang mit der pharmazeutischen Formulierung als separate Formulierung verabreicht werden kann.

Das Verabreichen von zwei unabhängigen Formulierungen, von denen eine den Wirkstoff und die andere den Lösungsstabilisator enthält, weist den Nachteil auf, dass der Patient mindestens zwei Tabletten oder Kapseln einnehmen muss, wobei immer das Risiko besteht, dass eine der beiden Formulierungen vergessen wird.

Enthält die pharmazeutische Formulierung sowohl die feste Dispersion als auch den Lösungsstabilisator in einer gemeinsamen Dosiseinheit, so ergibt sich das Problem, dass entsprechende Dosiseinheiten aufgrund der benötigten Menge an Lösungsstabilisator so schwer und damit groß werden können, dass sie nicht mehr geschluckt werden können, da mit steigender Menge an Wirkstoff auch die Menge an Lösungsstabilisator erhöht werden muss. So offenbart beispielsweise die WO 03/000294 A1 in ihrem allgemeinen Teil, dass es möglich ist, feste Dispersionen mit bis zu 80 Gew.-% Wirkstoff herzustellen. In den Beispielen 7 und 8 werden dann jedoch bei einer Wirkstoffbeladung von nur 25 Gew.-% bereits mindestens 20 Gew.-% Lösungsstabilisator benötigt.

Es besteht somit weiterhin ein Bedürfnis danach, eine pharmazeutische Formulierung der vorstehend beschriebenen Art zur Verfügung zu stellen, die eine möglichst hohe Wirkstoffbeladung aufweisen kann, wobei die amorphe Form des Wirkstoffs trotzdem ausreichend stabilisiert wird und genügend Lösungsstabilisator vorliegt, um ein Rekristallisieren des Wirkstoffs nach Auflösung beispielsweise im Magen zu verhindern.

Es wurde nun überraschend gefunden, dass eine entsprechende pharmazeutische Formulierung, die die vorstehenden Nachteile nicht aufweist, erhalten wird, wenn die feste Dispersion ein Polymer enthält, das gleichzeitig als Löslichkeitsverbesserer und Rekristallisationsinhibitor wirkt und die pharmazeutische Formulierung neben der festen Dispersion 3 bis weniger als 20 Gew.-% eines weiteren Polymers enthält, das als Lösungsstabilisator wirkt. Durch das Mischen der festen Dispersion mit einer geringen Menge eines zusätzlichen Lösungsstabilisators kann die Beladung der festen Dispersion mit dem Wirkstoff ganz erheblich erhöht werden, so dass die vorstehend beschriebenen Nachteile des Standes der Technik überwunden werden können.

Die vorliegende Erfindung betrifft somit eine pharmazeutische Formulierung, umfassend
a) eine feste Dispersion, die einen in amorpher Form in ein Polymer eingebetteten Wirkstoff enthält, und
b) unabhängig von der festen Dispersion ein externes Polymer als Lösungsstabilisator,
**dadurch gekennzeichnet, dass** die feste Dispersion ein Polymer enthält, das gleichzeitig als Löslichkeitsverbesserer und Rekristallisationsinhibitor wirkt und die pharmazeutische Formulierung 3 bis weniger als 20 Gew.-% externes Polymer enthält, bezogen auf das Gesamtgewicht aus fester Dispersion und externem Polymer.

Die erfindungsgemäße pharmazeutische Formulierung ist für praktisch jeden Wirkstoff geeignet, wobei solche Wirkstoffe bevorzugt sind, die in Wasser schwer löslich oder noch schlechter löslich sind. Unter in Wasser schwer löslichen Wirkstoffen werden vorliegend solche gemäß der Definition des Europäischen Arzneibuchs verstanden. Hierbei handelt es sich um Wirkstoffe, bei denen 1 Massenteil Substanz von 100-1000 Volumenteilen Lösungsmittel gelöst werden. Unter noch schlechter löslich werden solche Wirkstoffe verstanden, die mehr als 1000 Volumenteile Lösungsmittel für deren Auflösung benötigen. Entsprechende Wirkstoffe sind dem Fachmann bekannt und im Stand der Technik umfangreich beschrieben. Eine Auflistung findet sich beispielsweise in der WO 03/000294 A1. Erfindungsgemäß bevorzugt wird beispielsweise der Wirkstoff Celecoxib oder ein pharmazeutisch verträgliches Salz davon.

Die pharmazeutische Formulierung der vorliegenden Erfindung eignet sich beispielsweise für Wirkstoffe aus den folgenden Wirkstoffgruppen: Acetylcholinesterase-Inhibitoren, Aknemittel, Analgetika, Antiphlogistika, Antiadiposita, Antiarrhythmika, Antiasthmatika, Antidementiva, Antibiotika (darunter Penicilline, Cephalosporine, Carbapeneme, Glycopeptide, Lincosamide, Aminoglycoside, Tetracycline, Makrolide, Nitroimidazol-Derivate, Gyrasehemmer, Sulfonamide), Antidepressiva, Antidiabetika, Antiemetika, Antiepileptika, Antihistaminika / Antiallergika, Antihypertonika, (darunter zentral angreifende α2-Agonisten, ACE-Hemmer, α-Sympatholytika, β-Sympatholytika, AT-Rezeptorantagonisten, Ca-Antagonisten), Antikoagulatien, Antikonvulsiva, Antimykotika, Antiparkinsonmittel, Antiprotozoika, Antipsoriatika, Antipsychotika, Antirheumatika, Aldosteron-Antagonisten, Alkoholentwöhnungsmittel, Mittel zur Behandlung der benignen Prostatahyperplasie, Biphosphonate, Cannabinoide, Cholesterol-Resorptionshemmer, COX-1-Inhibitoren, COX-2-Inhibitor, unspzifische COX-Inhibitoren, Diuretika, Endothelin-Rezeptorantagonist, Glucocorticoide, H2-Rezeptorantagonisten, Immunsupressiva, Inkontinenzmittel, Hormone, Mittel zur Behandlung der Hyperphosphatämie, Immunmodulatoren, Immunsuppressiva, Kontrazeptiva, Koronarmittel, Lipase-Inhibitoren, Lipid-Senker, Magen-Darm-Mittel, Malariamittel, Mucolytika, Multikinase-Inhibitoren, Mittel zur Behandlung der Multiplen Sklerose, Muskelrelaxantien, α-Sympatomimetika, Neuroleptika, Osteoporosemittel, Biphosphonate, Mittel zur Behandlung von HIV, Parasympatholytika, Phosphodiesterase-Hemmer, Protonenpumpeninhibitoren, Mittel zur Behandlung von Prostata-CA, Psychostimulantien, Renin-Hemmer, Sedativa, Spasmolytika, Steroidhormine, Thrombinhemmer, Tranquilantien, Triptane, Tyrosinkinase-Inhibitoren, Virustatika, Vitamine, insbesondere fettlösliche, Zytostatika (darunter Antimetaboliten, Alkylierende SubstanzenTopoisomerase-Hemmstoffe, Mitosehemmstoffe, Anthracycline, Antiestrogene, Antiandrogene, Aromatasehemmer).

Beispielsweise können folgende Wirkstoffe oder ihre pharmazeutisch verträglichen Salze eingesetzt werden: Abacavir, Acamprosat, Acarbose, Acebutolol, Aceclofenac, Acemetacin, Acetazolamid, Acetophenazin, Acetylcystein, Aciclovir, Acipimox, Acitretin Alendronat, Alfentanil, Alfuzosin, Alimemazin, Aliskiren, Almagat, Almotriptan, Alphacalcidol, Alpha Tocopherylacetat, Alprazolam, Alprenolol, Amantadin, Ambrisentan, Amifostin, Amilorid, Aminoglutethimid, Aminopentamid, Amiodaron, Amisulprid, Amitryptilin, Amlodipin, Amoxicillin, Amphetamin, Amphotericin B, Ampicillin, Amprenavir, Amrinon, Amsacrin, Anastrozol, Anidulafungin, Aprepitant, Aripiprazole, Atazanavir, Atenolol, Atomoxetin, Atorvastatin, Atovaquon, Atrasentan, Atrolactamid, Atropin, Avanafil, Azapropazon, Azathioprin, Azelastin, Azithromycin, Azosemid, Beclemid, Beclometason, Bemetizid, Benazepril, Bendamustin, Bendroflumethiazid, Benperidol, Benserazid, Benzquinamid, Bervastatin, Betahistin, Betamethason, Betaxolol, Bezafibrat, Bicalutamid, Bifeprunox, Bifonazol, Biperiden, Bisoprolol, Bleomycin, Bosentan, Brimonidin, Bromazepam, Bromocriptin, Bromoprid, Bromperidol, Brotizolam, Budesonid, Budipin, Bumetamid, Bupranolol, Buprenorphin, Bupropion, Buspiron, Busulfan, Butizid, Butyrophenon, Cabergolin, Calcipotriol, Calcitriol, Camptothecin, Candesartan, Capecitabin, Captopril, Carbamazepin, Carbidopa, Carboplatin, Carmustin, Carphenazin, Carvedilol, Cefaclor, Cefadroxil, Cefalexin, Cefamandol, Cefazolin, Cefepim, Cefetametpivoxil, Cefixim, Cefodizim, Cefotaxim, Cefotiam, Cefoxitin, Cefpodoximproxetil, Cefsulodin, Ceftazidim, Ceftibuten,Ceftriaxon, Cefuroxim, Cefuroximaxetil, Celecoxib, Celiprolol, Cerivastatin, Cetirizin, Chinin, Chlorambucil, Chlordiazepoxid, Chloroquin, Chlorphenoxamin, Chlorpromazin, Chlorprothixen, Chlorprotixen, Ciclesonide, Ciclopirox-Olamin, Ciclosporin, Cilastatin Cilazapril, Cilostazol, Cimetidin, Cinacalcet, Ciprofloxacin, Cisaprid, Cisplatin, Citalopram, Cladribin, Clarithromycin, Clavulansäure, Clemastin, Clinafloxacin, Clindamycin, Clobazam, Clobazam, Clodronat, Clofibrat, Clomethiazol, Clomipramin, Clonazepam, Clonidin, Clopamid, Clopenthixol, Clopidogrel, Clospirazin, Clothiapin, Clotrimazol, Clozapin, Codein, Colecalciferol, Cortison, Cromoglycinsäure, Cyclophosphamid, Cyproteronacetat, Cytarabin, Dabigatran, Dacarbazin, Dactinomycin, Dalvastatin, Dapoxetin, Darifenacin, Dasatinib, Daunorubicin, Delavirdin, Denaverin, Desipramin, Desloratadin, Desmopressin, Desogestrel, Desogestrel, Dexamethason, Dexchlorpheniramin, Dexetimid, Diamorphin, Diazepam, Dibenzepin, Diclofenac, Didanosin, Dienogest, Diethazin, Diflunisal, Dihydrocodein, Diltiazem, Dimenhydrinat, Dimenhydrinat, Dimethadion, Dimetinden, Diphenhydramin, Diphenylhydantoin, Dipyridamol, Disopyramid, Docetaxel, Dolasetron, Domperidon, Donepezil, Doxazosin, Doxepin, Doxorubicin, Doxycycllin, Doxylamin, Drofenin, Dronedaron, Droperidol, Drospirenon, Droxidopa, Duloxetin, Dutasterid, Econazol, Efavirenz, Eletriptan, Emtricitabin, Enalapril, Enfuvirtid, Enoxacin, Enoximon, Entacapone, Epirubicin, Eplerone, Eprosartan, Erlotinib, Erythromycin, Escitalopram, Esomeprazol, Estradiol, Estramustin, Ethinylestradiol, Ethopropazin, Ethosuximid, Ethylbenzhydramin, Etidronat, Etofibrat, Etofyllinclofibrat, Etoposid, Etoricoxib, Exemestan, Ezetimib, 5-FU (Fluorouracil), Famciclovir, Famotidin, Famotidin, Felbamat, Felbamat, Felodipin, Fendilin, Fenetyllin, Fenofibrat, Fenoterol, Fentanyl, Fenticonazol, Fesoterodin, Fexofenadin, Finasterid, Flecainid, Fleroxacin, Fluanison, Fluconazol, Flucytosin, Fludarabin, Flumazenil, Flunisolid, Flunitrazepam, Fluocortolon, Fluoxetin, Flupentixol, Fluphenaminsäure, Fluphenazin, Flupirtin, Flurazepam, Flurbiprofen, Fluspirilen, Flutamid, Fluticason, Fluvastatin, Fluvoxamin, Folinsäure, Formoterol, Fosinopril, Fosphenytoin, Frovatriptan, Furosemid, Gabapentin, Galantamin, Gallopamil, Gatifloxacin, Gefitinib, Gemcitabin, Gemfibrozil, Geprofloxacin, Gestoden, Glatiramer, Glibenclamid, Glibornurid, Gliclazid, Glimepirid, Glipizid, Gliquidon, Glisoxepid, Glucosamin, Goserelin, Granisetron, Griseofulvin, Guanfacin, Halofantrin; Haloperidol; Hydrochlorothiazid, Hydrocodon; Hydrocortison, Hydromorphon; Hydroxyzin; Ibandronat, Ibuprofen, Idarubicin, Ifosfamid, Imatinib, Imipenem, Imipramin, Imiquimod, Indapamid, Indinavir, Indiplon, Indometacin, Ipratropium, Ipratropiumbromid, Irbesartan, Irinotecan, Isoconazol, Isotretinoin; Isradipin, Itraconazol, Ixabepilon, Ketoconazol, Ketoprofen, Ketotifen, Labetalol, Lacidipin, Lamivudin, Lamotrigin, Lansoprazol, Lapatinib, Leflunomid, Lenalidomide; Lercanidipin, Letrozol; Leuprorelin, Levetiracetam, Levocetirizin, Levodopa, Levofloxacin, Levomepromazin, Levomethadon, Levonorgesterl, Licofelone, Lidocain, □-Liponsäure, Lisinopril, Lisurid, Lofepramin, Lomustin, Lonazolac, Lopinavir, Loracarbef; Loratadin, Lorazepam, Lormetazepam, Lornoxicam, Losartan, Lovastatin, Manidipin, Maprotilin, Maraviroc, Mebeverin, Meclozin, Mefenaminsäure, Mefloquin, Mefrusid, Meloxicam, Melperon, Memantine, Mephenytoin, Mephobarbital, Mepindolol, Mercaptopurin, Meropenem, Mesalazin, Mesuximid, Metamizol, Metformin, Methadon, Methamphetamin, Methotrexat; Methoxypromazin, Methsuximid, Methyldopa, Methylphenidat, Methylprednisolon, Metipranolol, Metoclopramid, Metoprolol, Metronidazol, Mexiletin, Mianserin, Miconazol, Midazolam, Miglitol, Milrinon, Minocyclin, Mirtazapin, Misoprostol, Mitiglinid, Mitomycin, Mitoxantron; Mizolastin, Moclobemid, Modafinil, Moexipril, Mofegilin, Molindon, Molsidomin, Mometason, Montelukast, Moperon, Morphin, Mosapramin, Moxifloxacin, Moxonidin; Mycophenolsäure, Nadolol, Nalbuphin, Naloxon, Naltrexon, Naproxen, Naratriptan, Narcobarbital, Natamycin, Nateglinid, Na-Valproate, Naxagolid, Nebivolol, Nedocromil, Nefazodon, Nefopam, Nelfinavir, Nevirapin, Nicardipin, Nicorandil, Nicotin, Nifedipin, Nilutamid; Nilvadipin, Nimesulid, Nimetazepam, Nimodipin, Nimorazol, Nimustin, Nisoldipin, Nitrazepam, Nitrendipin, Nitrezepam, NitroglycerinTTS; Nizatidin, Norfloxacin, Norgestimat, Nortryptilin, Nystatin, Octreotid, Ofloxacin, Olanzapin, Olmesartan, Olpadronat, Olsalazin, Omeprazol, Ondansetron, Opipramol, Orlistat; Oseltamivir, Oxaceprol, Oxaflumazin, Oxaliplatin, Oxazepam, Oxcarbamazepin, Oxcarbazepin, Oxiconazol, Oxitropium; Oxitropiumbromid, Oxprenolol, Oxybutynin, Oxycodon; Paclitaxel, Paliperidone, Palonosetron, Pamidronat, Pantoprazol, Papaverin, Paracetamol, Paramethadion, Paroxetin, Pefloxacin, Pemetrexed, Pemolin, Penbutolol, Penciclovir, Penfluridol, Pentazocin, Pentostatin, Pentoxyfylin, Perazin, Pergolid, Perimethazin, Perindopril, Perphenazin, Pethidin, Phenacemid, Phenazon, Pheniramin, Phenobarbital, Phenotiazin, Phenprocoumon, Phensuximid, Phenylbutazon, Phenytoin, Pimecrolimus, Pimozid, Pindolol, Pioglitazon, Pipamperon, Piperacetazin, Piperacillin, Pirenzepin, Piretanid, Piribedil, Piritramid, Piroxicam, Pitavastatin, Pramipexol, Prasugrel; Pravastatin, Prazepam, Prednicarbat, Prednisolon, Prednison, Pregabalin, Primaquin, Primidon, Prochlorperazin, Progabid, Proguanil, Promazin, Promethazin, Propafenon, Propiverin, Propranolol, Propyphenazon, Prothipendyl, Pyrimethamin, Quetiapin, Quinapril, Rabeprazol; Racecadotril, Raloxifen, Ramelteon, Ramipril, Ranitidin, Ranolazin, Rasagilin, Reboxetin, Recuroniumbromid; Remacemid, Remifentanyl, Remoxiprid, Repaglinid, Retigabin, Ribavirin, Rifaximin, Rilmenidine, Riluzole, Rimonabant, Risedronat, Risperidon, Ritonavir, Rivaroxaban, Rivastigmin, Rizatriptan, Rofecoxib, Roflumilast, Ropinirol, Rosiglitazon, Rosuvastatin, Rotigotin, Roxatidinacetat, Roxithromycin, Rufinamid, Salmeterol, Saquinavir, Scopolamin, Selegilin, Sertindol, Sertraconazol, Sertralin, Sevelamer carbonate; Sevelamer, Sibutramin, Sildenafil, Simvastatin, Sirolimus, Sitagliptin, Solifenacin, Sorafenib; Sotalol, Sparfloxacin, Spiramycin, Spironolacton, Stavudin, Stiripentol, Strontiumranelat, Sufentanil, Sulbactam, Sulfacarbamid, Sulfadiazin, Sulfalen; Sulfamerazin, Sulfamethoxazol, Sulfasalazin; Sulforidazin, Sulpirid, Sultiam, Sumatriptan, Sunitinib, Tacrin, Tacrolimus, Tadalafil, Tamoxifen, Tamsulosin, Tazobactam; Teicoplanin, Telithromycin; Telmisartan, Temazepam, Temozolomid, Teniposid, Tenofovir, Tenoxicam, Terazosin, Terbinafin, Terfenadin,Tetrabenazin, Tetrazepam, Theophyllin, Thioguanin, Thioproperazin,Thioridazin, Thiotepa, Thiothixen, Thioxanthen, Tiagabin, Tianeptin, Tiaprid, Tiaprofensäure, Tibolon; Tilidin, Tiludronat, Timolol; Tinidazol; Tioconazol, Tiotropium, Tiropramid; Tizanidin, Tocainid, Tolbutamid, Tolterodin, Topiramat, Topotecan; Torasemid, Toremifen; Tramadol, Trandolapril, Tranylcypromin, Trazodon, Treosulfan, Tretinoin, Triamcinolon; Triamteren, Triazolam, Trichlormethiazid, Trifluoperazin, Trifluperidol, Triflupromazin, Triflusal, Trimetazidin, Trimethadion, Trimipramin, Tritoqualin, Trofosfamid, Tropicamid, Tropisetron; Trospiumchlorid, Urapidil, Valaciclovir, Valdecoxib, Valganciclovir, Valproat Natrium, Valproinsäure, Valsartan, Vancomycin; Vardenafil; Vareniclin, Vatalanib; Venlafaxin, Vigabatrin, Vildagliptin; Viloxazin; Vinblastin, Vincristin, Vinorelbin, Vitamine A,D,E,K, Voriconazol, Warfarin, Xipamid, Zafirliukast; Zalcitabin, Zaleplon, Zanamivir, Zidovudin, Ziprasidon, Zoledronat; Zolmitriptan, Zolpidem, Zonisamide, Zopiclon, Zotepin, Zuclopentihixol.

Die Wirkstoffe können einzeln oder in Kombination von zwei oder mehreren Wirkstoffen vorliegen. Ferner können die Wirkstoffe in ihrer freien Form, als pharmazeutisch verträgliche Salze oder als Cokristalle eingesetzt werden.

Erfindungsgemäß wird der Wirkstoff in einer festen Dispersion eingebettet, so dass er nach Einbettung in amorpher Form vorliegt. Unter "fester Dispersion" wird dabei eine Dispersion des amorphen Wirkstoffs in einer Polymermatrix verstanden. Vorzugsweise ist der amorphe Wirkstoff molekular dispers in der Polymermatrix verteilt. In diesem Fall handelt es sich bei der festen Dispersion um eine feste Lösung.

Die feste Dispersion enthält ein Polymer, das gleichzeitig als Löslichkeitsverbesserer und Rekristallisationsinhibitor wirkt. Dabei wird das Polymer als Löslichkeitsverbesserer bezeichnet, wenn es in der Lage ist, den Wirkstoff in seiner amorphen Form einzubetten. Hierdurch wird die Löslichkeit des ansonsten nur schwer löslichen Wirkstoffs verbessert. Als Rekristallisationsinhibitor wird das Polymer bezeichnet, weil es in der Lage ist, die amorphe Form des Wirkstoffs in der Polymermatrix zu stabilisieren und damit ein Rekristallisieren des Wirkstoffs beispielsweise während der Lagerung zu verhindern. Dementsprechend kann ein Fachmann ohne weiteres durch einfache Versuche geeignete Polymere, die sich sowohl für die Einbettung der amorphen Form des Wirkstoffs eignen als auch diese stabilisieren, auswählen. Geeignete Polymere sind auch im Stand der Technik beschrieben, beispielsweise der WO 03/000294 A1 und der EP-A-1 027 886.

Erfindungsgemäß bevorzugt wird das Polymer für die Einbettung des Wirkstoffs in amorpher Form in der festen Dispersion ausgewählt aus der Gruppe bestehend aus Eudragiten, insbesondere Eudragit E, Copovidon, Zuckeralkohole, Ammoniummethacrylat Copolymer, Poly(dimethylaminoethylmethacrylatcomethacrylat Ester), Poly(methylacrylatcomethylmethacrylatcomethacrylsäure), Poly(methacrylsäurecomethylmethacrylat) 1:2, Hydroxypropylcellulose, Ethylcellulose, Celluloseacetatbutyrat, Celluloseacetatphthalat, Poly(ethylenoxid), Poly(ethylenglycol), Poly(vinylpyrrolidon), Poly(vinylacetat), Hydroxypropylmethylcellulosephthalat, Polyvinylpyrrolidoncovinylacetat, Hydroxypropylmethylcellulose, Hydroxypropylmethylcelluloseacetatsuccinat, Poly(lactidcoglycolid), Polyvinylalkohol, Chitosanlactat, Pectin, Carbomer, Polycarbophil, Poly(ethylencovinylacetat), Polyethylen, Poly(vinylacetatcomethacrylsäure), Polycaprolacton, Carnaubawachs, Ethylenvinylacetat Copolymer, Glycerolpalmitostearat, Stärke, Maltodextrin, Isomalt und Polyvinylalkoholpolyethylenglycol Copolymer. Die Polymere können alleine oder als Mischung von zwei oder mehr Polymeren verwendet werden. Vorzugsweise enthält die feste Dispersion jedoch nur ein Polymer.

In einer besonders bevorzugten Ausführungsform besteht die feste Dispersion aus dem Wirkstoff oder einem pharmazeutisch verträglichen Salz davon und einem Polymer. Zusätzliche Polymere und/oder Hilfsstoffe wie Weichmacher, Füllmittel, etc. würden demgegenüber die Rekristallisationsneigung des amorphen Wirkstoffs erhöhen. Die vorliegende Erfindung ermöglicht somit einen Verzicht auf entsprechende, sonst übliche Zusätze und stellt dementsprechend besonders lagerstabile Formulierungen zur Verfügung. Soweit entsprechende Zusätze benötigt oder gewünscht werden, können diese mit dem externen Polymer gemischt vorliegen, ohne hierdurch die amorphe Form des Wirkstoffs zu destabilisieren.

In der erfindungsgemäßen pharmazeutischen Formulierung liegt die feste Dispersion, die einen in amorpher Form in ein Polymer eingebetteten Wirkstoff enthält, zusammen mit einem weiteren Polymer vor, das als Lösungsstabilisator wirkt. Das zusätzliche Polymer bildet dabei keinen Bestandteil der festen Dispersion und wird daher auch als externes Polymer bezeichnet. Die beiden Komponenten können beispielsweise einfach miteinander vermischt vorliegen oder beispielsweise in Tabletten in zwei voneinander getrennten Räumen, in Form eines Kerns mit einem Überzug, wobei beispielsweise der Kern aus der festen Dispersion und der Überzug aus dem externen Polymer oder umgekehrt gebildet sein kann. Alternativ kann die feste Dispersion beispielsweise gemahlen, mit dem externen Polymer gemischt und als Mischung z.B. in Kapseln gefüllt oder in Tabletten verpresst werden. Das externe Polymer darf nur nicht auf molekularer Ebene mit der festen Dispersion gemischt sein.

Als Lösungsstabilisator für das externe Polymer eignen sich beispielsweise gelbildende Polymere, wie sie dem Fachmann bekannt sind. Das externe Polymer kann insbesondere ausgewählt sein aus der Gruppe bestehend aus Cellulosederivaten, insbesondere Methylencellulose (MC), Hydroxypropylcellulose (HPC), Hydroxypropylmethylcellulose (HPMC), Natriumcarboxymethylcellulose (NaCMC), Carrageenan, Xanthan, Tragant, Johannisbrotkernmehl, Guarkernmehl, Copolymere der Methacrylsäure, Galactomannane, Alginate, Stärke, Modifizierte Stärke, Agar-Agar, Gummi arabicum, Tarakernmehl und Pektin. Als externes Polymer kann ein einzelnes Polymer oder eine Mischung von zwei oder mehr Polymeren eingesetzt werden. Ein besonders bevorzugtes externes Polymer ist Hydroxypropylmethylcellulose (HPMC).

In einer besonders bevorzugten Ausführungsform enthält die pharmazeutische Formulierung der vorliegenden Erfindung Eudragit, insbesondere Eudragit E als Polymer der festen Dispersion und HPMC als externes Polymer.

Die Kombination aus einem in amorpher Form in ein Polymer eingebetteten Wirkstoff mit einer geringen Menge eines externen Polymers als Lösungsstabilisator ermöglicht es, die feste Dispersion mit einer großen Menge Wirkstoff zu beladen. Die in der erfindungsgemäßen pharmazeutischen Formulierung enthaltene feste Dispersion enthält somit vorzugsweise mindestens 30 Gew.-% Wirkstoff bezogen auf das Gesamtgewicht der festen Dispersion. Insbesondere enthält die feste Dispersion mindestens 40 Gew.-%, besonders bevorzugt 45-55 Gew.-% Wirkstoff, bezogen auf das Gesamtgewicht der festen Dispersion. Es können jedoch auch geringere Mengen Wirkstoff eingesetzt werden, beispielsweise mindestens 5 Gew.-%, mindestens 10 Gew.-% oder mindestens 20 Gew.-%, jeweils bezogen auf das Gesamtgewicht der festen Dispersion.

Durch die Kombination aus der festen Dispersion aus in amorpher Form eingebettetem Wirkstoff und Polymer, das gleichzeitig als Löslichkeitsverbesserer und Rekristallisationsinhibitor wirkt, benötigt die erfindungsgemäße pharmazeutische Formulierung nur eine geringe Menge externes Polymer, um ein Rekristallisieren des Wirkstoffs aus einer übersättigten Lösung zu verhindern. Die erfindungsgemäße pharmazeutische Formulierung enthält somit nur 3 bis weniger als 20 Gew.-% externes Polymer, bezogen auf das Gesamtgewicht aus fester Dispersion und externem Polymer. Insbesondere enthält die Formulierung 3 bis 18 Gew.-%, beispielsweise 3 bis 15 Gew.-% des externen Polymers, bezogen auf das Gesamtgewicht aus fester Dispersion und externem Polymer. Vorzugsweise enthält die pharmazeutische Formulierung der vorliegenden Erfindung 5-15 Gew.-%, besonders bevorzugt 8-12 Gew.-% des externen Polymers, bezogen auf das Gesamtgewicht aus fester Dispersion und externem Polymer.

Die erfindungsgemäße pharmazeutische Formulierung kann als an sich bekanntes Arzneimittel, insbesondere feste Arzneiform vorliegen. Diese kann beispielsweise ausgewählt sein aus Tabletten, orodispersiblen Tabletten, Schmelztabletten, Pellets, Hartgelatinekapseln, Dragees, Granulaten, etc. Pellets und Granulate können beispielsweise in Sachets oder Stick-Packs abgefüllt vorliegen. Es sind aber auch flüssige Arzneiformen wie Suspensionen umfasst.

Erfindungsgemäß bevorzugt sind Festdosierungsformen, wie Pellets, Kapseln und Tabletten. Diese können beschichtet oder unbeschichtet sein. Tabletten können beispielsweise durch Trockenkompaktierung oder Direktverpressen der zerkleinerten, insbesondere gemahlenen festen Dispersion in Mischung mit dem externen Polymer hergestellt werden.

Pellets können beispielsweise durch Schneiden von Strängen beim Verlassen eines Extruders und anschließendes Abkühlen hergestellt werden. Dazu eignet sich insbesondere ein hinter den Extruder geschalteter Mikropelletizer. Zur Herstellung von Granulaten eignet sich beispielsweise ein hinter einem Extruder und ein Kühlband geschalteter Granulator, der die erstarrten Stränge in Granulate schneidet.

Zur Herstellung von Kapseln kann die feste Dispersion gemahlen und mit dem externen Polymer sowie gegebenenfalls weiteren Hilfsstoffen gemischt und in Kapseln, beispielsweise Gelatinekapseln, abgefüllt werden.

Die Formulierung der festen Dispersion zu Tabletten erfolgt mit üblichen pharmazeutischen Hilfs- und Zusatzstoffen, wobei das externe Polymer zur Lösungsstabilisierung ebenfalls zugesetzt wird. Weitere geeignete Hilfsstoffe sind in der Regel Füllstoffe, Bindemittel, Sprengmittel, Fließregulierungsmittel, Schmiermittel, Geschmacksstoffe sowie gegebenenfalls weitere Zusatzstoffe.

Die erfindungsgemäßen pharmazeutischen Formulierungen können beispielsweise 0-90 Gew.-% Hilfsstoffe enthalten.

Der Gehalt an Sprengmitteln kann im Bereich von 1-40 Gew.-%, vorzugsweise 20-30 Gew.-% liegen, abhängig vom verwendeten Sprengmittel, der Füllstoffe und der übrigen Zusatzstoffe.

Der Gehalt an Schmiermittel ist in der Regel im Bereich von 0,1-4 Gew.-%.

Die vorstehend genannten Hilfsstoffmengen beziehen sich jeweils auf das Gesamtgewicht der pharmazeutischen Formulierung.

Als Füllstoffe können eine oder mehrere Verbindungen verwendet werden, wie z.B. mikrokristalline Cellulose, Stärke, Cellulosepulver, Lactose, insbesondere sprühgetrocknete Lactose, Glucose, Mannitol und Sorbitol.

Geeignete Sprengmittel sind Stärke, insbesondere Maisstärke, Alginsäure und deren Salze und Derivate, wie Calciumalginat und Natriumalginat, Natriumcarboxymethylcellulose, Polyacrylsäure, quervernetztes Polyvinylpyrrolidon, vernetzte Natriumcarboxymethylcellulose, quervernetzte Natriumcarboxymethylstärke, niedrigsubstituierte Natriumcarboxymethylcellulose, Natriumhydrogencarbonat und Magnesiumperoxid. Geeignete Schmiermittel sind beispielsweise Magnesiumstearat, Calciumbehenat, Glycerinmonostearat, Stearinsäure, hydrierte Pflanzenfette, PEG 4000, Natriumdodecylsulfat, Magnesiumdodecylsulfat und Talcum. Als Fließregulierungsmittel eignet sich beispielsweise hochdisperses Siliciumdioxid.

Liegt die erfindungsgemäße pharmazeutische Formulierung beispielsweise als Tablette oder Pellet vor, kann sie mit einem oder mehreren Beschichtungsmitteln mit einer oder mehreren Schichten filmbeschichtet sein. Verwendbare Beschichtungsmittel sind beispielsweise Filmbildner, wie Cellulosederivate (z.B. MC, HEC, HPC, HPMC, NaCMC), Methacrylate, Copovidon, PVP, Celluloseacetatphthalat, Hyroxypropylmethylcellulosephthalat, Carboxymethylethylcellulose, Polyvinylacetatphthalat, Schellack, Methacrylsäureester und Ethylcellulose.

Falls erforderlich, können den Filmen weitere Hilfsstoffe wie z.B. Weichmacher zugesetzt werden.

Falls für die Beschichtung Wasser eingesetzt wird, kann dies zu einer Kristallisation des Wirkstoffs in der festen Dispersion führen oder diese beschleunigen. In diesem Fall kann es wünschenswert sein, die Beschichtung als "Dry Coating" aufzubringen oder eine Zwischenschicht vorzusehen, die die feste Dispersion vor einer Berührung mit dem Lösungsmittel während der Beschichtung schützt. Gleiches gilt, wenn die äußere Beschichtung eine Substanz wie einen Weichmacher enthält, die sich nachteilig auf die Stabilität der amorphen Form des Wirkstoffs auswirken kann.

Das Tablettengewicht ist nicht besonders eingeschränkt, übliche Tabletten sind 100 bis 800 mg, z.B. 300 bis 400 mg.

Die Herstellung fester Dispersionen, die für die erfindungsgemäße pharmazeutische Formulierung geeignet sind, ist dem Fachmann bekannt. Verschiedene Verfahren sind beispielsweise in der WO 03/000294 A1 beschrieben. Vorzugsweise wird die feste Dispersion allerdings durch Schmelzextrusion hergestellt. Zur Herstellung eines entsprechenden Extrudats wird der Wirkstoff mit dem Polymer gemischt. Diese Mischung wird in den Extruder eingebracht und dort zu einer homogenen Schmelze verarbeitet. Hierzu kann das Schneckenprofil beispielsweise Knetblöcke beinhalten, da die durch die Knetblöcke erzeugten Scherkräfte zum Aufschmelzen der Mischung beitragen. Da der Wirkstoff im Polymer löslich ist, erfolgt das Aufschmelzen vorzugsweise bei einer Temperatur unterhalb des Schmelzpunkts des Wirkstoffs. Besonders bevorzugt erfolgt das Aufschmelzen bei einer Temperatur, die 0,1-5 °C oberhalb der Glasübergangstemperatur oder des Schmelzpunkts des Polymers und 0,1-5 °C unterhalb des Schmelzpunkts des Wirkstoffs liegt.

Über die Temperatur der letzten Zylinder vor der Düsenplatte kann die Viskosität der Schmelze reguliert werden. Dabei gibt es für jedes System eine Mindesttemperatur, da die Düse sonst verstopft. Die Extrusion erfolgt hierbei vorzugsweise bei einem Auslassdruck von ca. 20 bar bis ca. 100 bar, insbesondere von ca. 20 bar bis ca. 50 bar.

Damit die Extrudate nicht zu einer einzigen Masse zusammenkleben sondern als getrennte Stränge erstarren ist es darüber hinaus vorzuziehen, dass das Extrudat nicht klebrig ist.

Hierzu sollte die feste Dispersion eine Glasübergangstemperatur oberhalb von 18 °C aufweisen.

Das erhaltene Extrudat liegt typischerweise in unterschiedlich langen Strängen vor, die nach dem Austritt aus dem Extruder nach dem Abkühlen zufällig gebrochen werden. Diese können dann weiter beispielsweise mit einer Mühle, z.B. einer Zentrifugalmühle zerkleinert werden, um Fraktionen bestimmter Partikelgröße zu erhalten. Je nach Mahlvorgang kann man dabei Partikel unterschiedlicher Größe herstellen. Typischerweise liegt die Partikelgröße unterhalb 500 µm. Dies entspricht gleichzeitig der Partikelgröße der am meisten eingesetzten Hilfsstoffe zur Herstellung fester Arzneiformen. Somit wird ein besseres Mischen des gemahlenen Extrudats mit den Hilfsstoffen ermöglicht.

Die Schmelze verlässt den Extruder in Form von Strängen unterschiedlicher Länge, die als solche noch keine Arzneiform darstellen. Um die Schmelze zu einer gleichmäßig dosierten Arzneiform zu verarbeiten, muss das Extrudat zunächst zerkleinert, beispielsweise in einer Mühle gemahlen werden. Der Mahlprozess hat dabei auf die amorphe, unterkühlte/glasartige Schmelze keinen Einfluss, so dass sich keine Kristallkerne bilden und das amorphe System stabil bleibt.

Die Schmelzextrusion hat gegenüber anderen bekannten Herstellungsverfahren den Vorteil, dass durch eine intensive Homogenisierung feste Lösungen erhalten werden können. Außerdem kann mit relativ hochviskosen Polymerschmelzen gearbeitet werden. Entsprechende Polymere mit einer relativ hohen Glasübergangstemperatur eignen sich besonders als Rekristallisationsinhibitoren. Schließlich kann auf den Einsatz von Lösungsmitteln bei der Schmelzextrusion vollständig verzichtet werden, so dass ein aufwändiges Entfernen von Lösungsmittelrückständen nicht erforderlich ist.
- Figur 1:: Freisetzung erfindungsgemäßes (transparentes) Eudragit E-Celecoxib (CEL)-Extrudate (50% CEL); Paddle, 0,1N HCl, 250nm, im Vergleich zu nicht erfindungsgemäßem, kristallinem (opaquen) Extrudat und einer physikalischen Mischung aus CEL und Eudragit E.
- Figur 2:: Rekristallisationsinhibition durch Zusatz von 10% HPMC zum Freisetzungsmedium; Paddle, 0,1N HCl, 250nm. Die unteren Kurven stellen die Freisetzung des reinen gemahlenen Extrudats dar (50% Celecoxib, 50% Eudragit E); die oberen Kurven die Freisetzung des reinen gemahlenen Extrudats (50% Celecoxib, 50% Eudragit E) in einer Lösung, die 10% (bezogen auf die Extrudat-Einwaage) HPMC enthält. Dargestellt sind jeweils zwei Kurven, also n=2.
- Figur 3:: Lagerstabilität Eudragit E-CEL-Extrudate (50% CEL) bei RT / 60% r.F.
- Figur 4:: Stabilität Eudragit E-Celecoxib-Extrudate (50% CEL) bei mechanischer Einwirkung in einer Mühle auf das Extrudat.

Die vorliegende Erfindung wird nachfolgend anhand von Beispielen erläutert, die jedoch nicht einschränkend sein sollen.

### Beispiele:

### Beispiel 1:

### Schme/zextrusion

Für die Schmelzextrusion wurden 50% Gew.-% Eudragit E und 50 Gew.-% Celecoxib in einem Turbula-Mischer gemischt und über die Dosiereinheit in den Extruder dosiert. Die Extrusion erfolgte bei einem Auslassdruck von 20 bis 50 bar. Die Zylinder wurden vor dem Einsatz mehrere Stunden vorgewärmt.

Unter den gewählten Bedingungen wurden transparente Extrudate in Form von Strängen mit einem Durchmesser von ca. 1 mm erhalten, welche bei Raumtemperatur abgekühlt und gemahlen wurden.

### Beispiel 2:

### Löslichkeitsverbesserung

Die Freisetzung des Wirkstoffs aus den Extrudaten aus Beispiel 1 wurde in 900 mL 0,1 N HCl mit einer Paddle-Apparatur gemessen. Um zu sehen, ob die Lösungsgeschwindigkeit durch Herstellung einer festen Lösung verbessert werden konnte, wurden neben dem transparenten Extrudat auch ein opaques (d.h. kristallinen Wirkstoff enthaltendes) Extrudat und die physikalische Mischung aus Wirkstoff und Polymer freigesetzt.

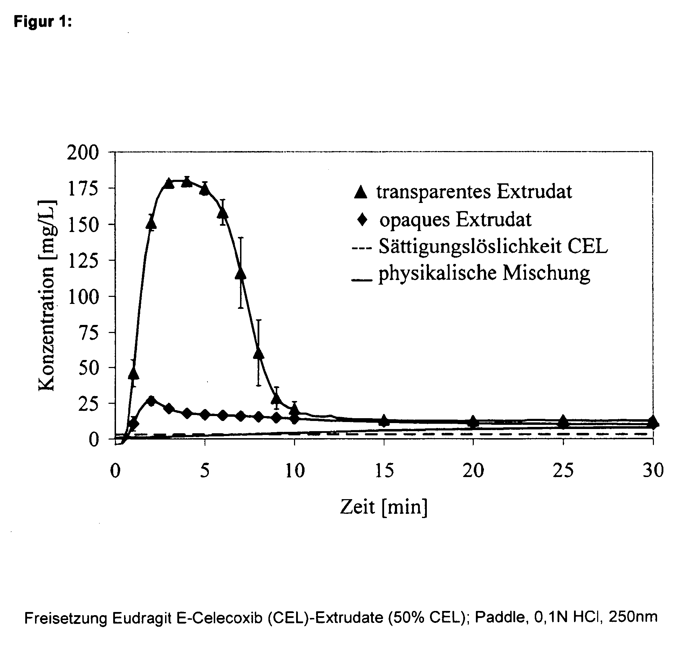

Die in vitro Freisetzungsversuche ergaben, dass innerhalb 5 Minuten eine 33-fache Übersättigung stattfindet. Liegen Kristalle im Extrudat vor, kommt es nur zu einer geringeren Übersättigung. Die Ergebnisse sind in Figur 1 graphisch dargestellt.

### Beispiel 3:

### Rekristallisationsinhibition

Die in vitro Freisetzungsversuche haben gezeigt, dass der Wirkstoff nach ca. 10 Minuten wieder ausfällt. Damit der Wirkstoff vom Körper resorbiert werden kann, muss er aber gelöst vorliegen. Deshalb wurde versucht, den Wirkstoff möglichst lange in Lösung zu halten.

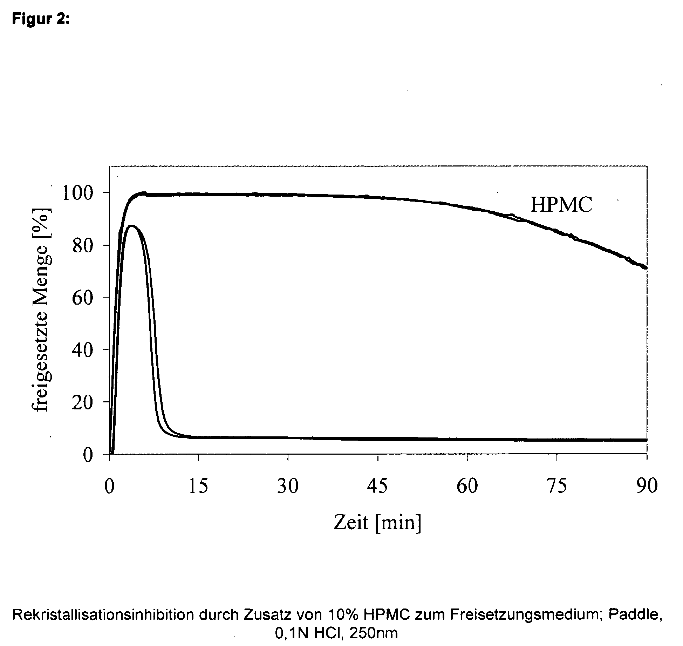

Dazu wurden 10% HPMC zum Freisetzungsmedium gegeben und die Probe aus Beispiel 2 erneut freigesetzt. Der Wirkstoff wurde in 5 bis 10 Minuten vollständig freigesetzt und die übersättigte Lösung wurde über einen Zeitraum von ca. 60 Minuten stabilisiert. Das Ergebnis ist in Figur 2 dargestellt.

### Beispiel 4:

### Stabilität der Extrudate

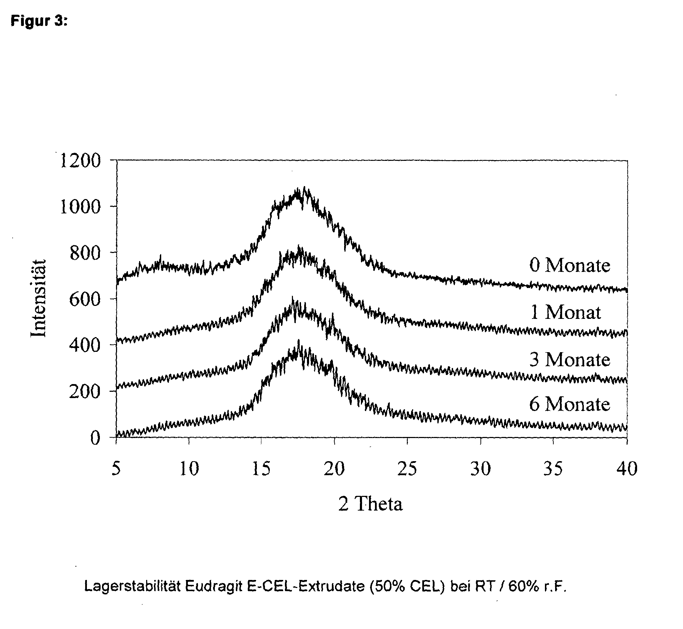

Die Extrudate aus Beispiel 1 wurden 6-12 Monate bei Raumtemperatur und bei Raumtemperatur / 60% relativer Feuchte gelagert, um die Stabilität der amorphen Systeme zu untersuchen. Der Versuch zeigte, dass die transparenten Extrudate lagerstabil sind und nicht rekristallisieren. Die Ergebnisse sind in Figur 3 wiedergegeben.

### Beispiel 5:

### Einfluss von mechanischem Energieeintrag durch Mahlen

Die Extrudatstränge aus Beispiel 1 wurden mit einer Zentrifugalmühle in Partikel <500µm gemahlen, um sie weiter zu Tabletten, Kapseln etc. verarbeiten zu können. Da ein mechanischer Energieeintrag sich negativ auf das amorphe System auswirken kann, indem sich Kristallkeime bilden, wurde mittels Röntgendiffraktometrie überprüft, ob sich nach dem Mahlen Kristallkeime gebildet haben.

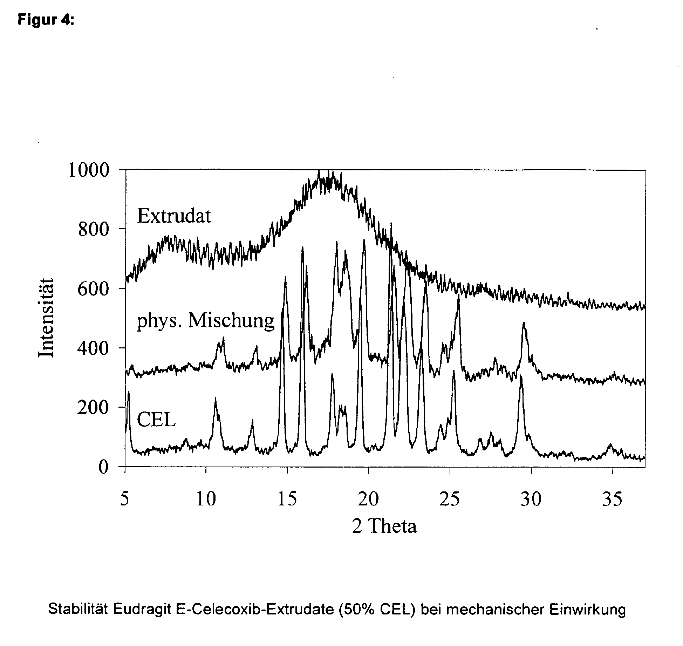

Als Ergebnis konnte festgestellt werden, dass das Mahlen keinen Einfluss auf den amorphen Zustand hat und die Extrudate auch nach der Verarbeitung zu Pulver amorphe Systeme darstellen, da keine Banden des kristallinen Celecoxibs detektierbar sind. Das Ergebnis ist in Figur 4 wiedergegeben.

## Patentansprüche

1. Pharmazeutische Formulierung, umfassend
a) eine feste Dispersion, die einen in amorpher Form in ein Polymer eingebetteten Wirkstoff enthält, und
b) unabhängig von der festen Dispersion ein externes Polymer als Lösungsstabilisator,
**dadurch gekennzeichnet, dass** die feste Dispersion ein Polymer enthält, das gleichzeitig als Löslichkeitsverbesserer und Rekristallisationsinhibitor wirkt und die pharmazeutische Formulierung 3 bis weniger als 20 Gew.-% externes Polymer enthält, bezogen auf das Gesamtgewicht aus fester Dispersion und externem Polymer.

2. Pharmazeutische Formulierung nach Anspruch 1, **dadurch gekennzeichnet, dass** der Wirkstoff in Wasser schwer löslich oder schlechter löslich als schwer löslich ist.

3. Pharmazeutische Formulierung nach einem der vorhergehenden Ansprüche,
**dadurch gekennzeichnet, dass** der Wirkstoff Celecoxib oder ein pharmazeutisch verträgliches Salz davon ist.

4. Pharmazeutische Formulierung nach einem der vorhergehenden Ansprüche,
**dadurch gekennzeichnet, dass** das Polymer der festen Dispersion ausgewählt ist aus der Gruppe bestehend aus Eudragiten, insbesondere Eudragit E, Copovidon, Zuckeralkohole, Ammoniummethacrylat Copolymer, Poly(dimethylaminoethylmethacrylatcomethacrylat Ester), Poly(methylacrylatcomethylmethacrylatcomethacrylsäure), Poly(methacrylsäurecomethylmethacrylat) 1:2, Hydroxypropylcellulose, Ethylcellulose, Celluloseacetatbutyrat, Celluloseacetatphthalat, Poly(ethylenoxid), Poly(ethylenglycol), Poly(vinylpyrrolidon), Poly(vinylacetat), Hydroxypropylmethylcellulosephthalat, Polyvinylpyrrolidoncovinylacetat, Hydroxypropylmethylcellulose, Hydroxypropylmethylcelluloseacetatsuccinat, Poly(lactidcoglycolid), Polyvinylalkohol, Chitosanlactat, Pectin, Carbomer, Polycarbophil, Poly(ethylencovinylacetat), Polyethylen, Poly(vinylacetatcomethacrylsäure), Polycaprolacton, Carnaubawachs, Ethylenvinylacetat Copolymer, Glycerolpalmitostearat, Stärke, Maltodextrin, Isomalt und Polyvinylalkoholpolyethylenglycol Copolymer.

5. Pharmazeutische Formulierung nach einem der vorhergehenden Ansprüche,
**dadurch gekennzeichnet, dass** das externe Polymer ausgewählt ist aus der Gruppe bestehend aus Cellulosederivaten, Carrageenan, Xanthan, Tragant, Johannisbrotkernmehl, Guarkernmehl, Copolymere der Methacrylsäure, Galactomannane, Alginate, Stärke, Modifizierte Stärke, Agar-Agar, Gummi arabicum, Tarakernmehl und Pektin.

6. Pharmazeutische Formulierung nach einem der vorhergehenden Ansprüche,
**dadurch gekennzeichnet, dass** das externe Polymer Hydroxypropylmethylcellulose ist.

7. Pharmazeutische Formulierung nach einem der vorhergehenden Ansprüche,
**dadurch gekennzeichnet, dass** die feste Dispersion mindestens 30 Gew.-%, insbesondere mindestens 40 Gew.-%, vorzugsweise 45-55 Gew.-% Wirkstoff enthält, bezogen auf das Gesamtgewicht der festen Dispersion.

8. Pharmazeutische Formulierung nach einem der vorhergehenden Ansprüche,
**dadurch gekennzeichnet, dass** sie 5-15 Gew.-%, vorzugsweise 8-12 Gew.-% externes Polymer enthält, bezogen auf das Gesamtgewicht der festen Dispersion.

9. Pharmazeutische Formulierung nach einem der vorhergehenden Ansprüche,
**dadurch gekennzeichnet, dass** sie in der festen Dispersion nur ein Polymer enthält.

10. Pharmazeutische Formulierung nach einem der vorhergehenden Ansprüche,
**dadurch gekennzeichnet, dass** die feste Dispersion durch Schmelzextrusion erhältlich ist.

11. Pharmazeutische Formulierung nach Anspruch 10, **dadurch gekennzeichnet, dass** die feste Dispersion eine Glasübergangstemperatur von >18 °C aufweist.

12. Verfahren zur Herstellung einer pharmazeutischen Formulierung nach einem der Ansprüche 1-11, umfassend die Schritte:
- Aufschmelzen und Mischen des Polymers, das gleichzeitig als Löslichkeitsverbesserer und Rekristallisationsinhibitor wirkt, und des Wirkstoffs,
- Abkühlen der Schmelze um eine feste Dispersion zu erhalten, die einen in amorpher Form in das Polymer eingebetteten Wirkstoff enthält,
- Zerkleinern der festen Dispersion,
- Mischen der festen Dispersion mit einem externen Polymer als Lösungsstabilisator und
- Herstellen der pharmazeutischen Formulierung aus dem Gemisch.

13. Verfahren nach Anspruch 12, wobei die feste Dispersion durch Schmelzextrusion erhalten wird.

14. Verfahren nach Anspruch 13, wobei das Aufschmelzen bei einer Temperatur unterhalb des Schmelzpunkts des Wirkstoffs erfolgt.

15. Verfahren nach Anspruch 14, wobei das Aufschmelzen bei einer Temperatur erfolgt, die 0,1-5 °C oberhalb der Glasübergangstemperatur oder des Schmelzpunkts des Polymers und 0,1-5 °C unterhalb des Schmelzpunkts des Wirkstoffs liegt.
